# EUROPEAN PATENT SPECIFICATION

(11) **EP 2 478 824 B1**
(45) Date of publication and mention of the grant of the patent: **26.12.2018**
(21) Application number: 10824789.1
(22) Date of filing: 05.10.2010
(51) Int. Cl.: A61B 1/04, G02B 23/24, A61B 1/00, A61B 5/00

(54) **ENDOSCOPE AND WIRELESS ENDOSCOPE SYSTEM**
ENDOSKOP UND DRAHTLOSES ENDOSKOPSYSTEM
ENDOSCOPE ET SYSTÈME D'ENDOSCOPE SANS FIL

(30) Priority: 21.10.2009 JP 2009242383
(43) Date of publication of application: 25.07.2012
(73) Proprietor: Olympus Corporation, Tokyo 192-8507 (JP)
(72) Inventor: KAWASAKI, Shinya, Hachioji-shi, Tokyo 192-8507 (JP); HONDA, Takemitsu, Hachioji-shi, Tokyo 192-8507 (JP)
(74) Representative: Gunzelmann, Rainer
(86) International application number: PCT/JP2010/067420
(87) International publication number: WO 2011/048940

(56) References cited:
- EP-A1- 2 042 077
- JP-A- 2006 295 564
- JP-A- 2008 085 505
- JP-A- 2008 085 505
- JP-A- 2009 189 663
- US-A1- 2005 261 552
- US-A1- 2009 209 810

## Description

### TECHNICAL FIELD

The present invention, which is defined in independent claim 1 and the dependent claims 2 - 8, relates to an endoscope scope that wirelessly transmits moving image data and still image data generated by photographing a subject. More specifically, the endoscope scope of the present invention is configured to transmit moving image data and still image data to a processor that displays a moving image and a still image.

### BACKGROUND ART

When a user gives a freeze instruction (or release instruction) at a notable part from an endoscope scope (hereinafter referred to as a "scope") in an endoscope system in which the scope and a processor are connected by a wire, a still image is generated and stored in a processor side. For example, as shown in FIG. 1 of Patent Document 1, after imaging data generated by a scope is sent as an analog signal to a processor, a still image is generated through a digital process in an analog/digital (A/D) conversion unit of the processor and the like, and the generated still image is stored.

Meanwhile, also in a wireless endoscope system in which a scope side and a processor side are wirelessly connected and a moving image photographed by the scope side is transmitted to the processor side in real time, a user may give a freeze instruction while viewing the moving image. When a still image is temporarily stored in the scope side by the freeze instruction, it is preferable for the stored still image to be transmitted to the processor side without being discarded such that the user can perform a complete medical checkup and the like.

Patent Document 2 discloses an electronic endoscope provided with an FM circuit and a modulation circuit, wherein electric power is supplied to the electronic endoscope from a battery that is connected to a voltage detection circuit. The voltage detector detects a voltage of the battery and inputs the detected voltage to a CPU that determines whether or not the voltage of the battery is equal to or lower than a predetermined value. If the CPU determines that the voltage is equal to or lower than the predetermined value, the CPU causes the SSB modulation circuit, which consumes less power, to modulate image data.

Patent Document 3 discloses a communication method for a wireless network including a step of transmitting a communication request packet from a source terminal to a destination terminal in an area capable of direct communication, and a step of sending a communication permission packet back to the source terminal from the destination terminal. Terminals other than the source terminal and destination terminal stop the transmission of the wireless signal temporarily when the communication request packet is received and when the communication permission packet is received.

EP 2042077 discloses an endoscope scope comprising a generation unit that photographs a subject and generates moving and still image data; a reception unit that receives a transmission instruction for the still image data; a transmission unit that wirelessly transmits the still image data, the moving image data and instruction information; a voltage detection unit that detects battery voltage; and a transmission control unit that causes the transmission unit to transmit the still image data when receiving the transmission instruction.

### CITATION LIST

Patent Document 1: Japanese Unexamined Patent Application, First Publication, No. 2008-264313
Patent Document 2: European Patent Application Publication No. 2 042 077 A1
Patent Document 3: Japanese Patent Application Publication No. 2008 085505 A

### SUMMARY OF INVENTION

### Problems to be solved by the Invention

In a system that transmits still image data while continuously transmitting moving image data such as the above-mentioned wireless endoscope system, a data packet needs to be transmitted in as short a time as possible such that a scope can be driven by a battery. Since moving image data requires a real-time processing, the number of packets retransmissions is small. Also, a moving image data packet that has not been transmitted in a predetermined amount of time is discarded, and a new packet is transmitted. On the other hand, still image data does not require the real-time processing. Thus, a packet is discarded after a long time, and the number of retransmissions of the still image data packet is set to be large. When interference is caused by a nearby wireless device based on a wireless standard such as Institute of Electrical and Electronics Engineers (IEEE) 802.11b, a transmission time lengthens due to data retransmission, and current consumption increases, lowering the remaining capacity of a battery.

The present invention provides an endoscope scope capable of reducing a decrease in the capacity of a battery caused by retransmission of a still image in the endoscope scope, and transmitting still image data.

### Means for Solving the Problems

An endoscope scope includes the features of claim 1. An endoscope scope may include: a generation unit that photographs a subject and generates moving image data and still image data; a reception unit that receives a transmission instruction for the still image data; a transmission unit that wirelessly transmits the still image data, the moving image data, and instruction information to instruct prohibition of wireless transmission by another wireless device; and a transmission control unit that causes the instruction information to be transmitted and then the still image data to be transmitted when receiving the transmission instruction.

The endoscope scope further includes a voltage detection unit that detects battery voltage. The transmission control unit receives the transmission instruction, and causes the instruction information to be transmitted and then the still image data to be transmitted when the battery voltage detected by the voltage detection unit is less than a predetermined value.

The reception unit may further receive a power-off instruction. The transmission control unit may receive the transmission instruction and the power-off instruction, and cause the instruction information to be transmitted and then the still image data to be transmitted when the still image data remains in the endoscope scope.

The endoscope scope may further include: a device detection unit that detects the other wireless device. The transmission control unit may receive the transmission instruction, and cause the instruction information to be transmitted and then the still image data to be transmitted when the other wireless device is detected.

The endoscope scope may further include: a voltage detection unit that detects battery voltage; and a notification unit that notifies a processor that the battery voltage is low when the battery voltage detected by the voltage detection unit becomes less than a predetermined value. The transmission control unit may cause the instruction information to be transmitted and then the still image data to be transmitted when the transmission control unit is notified that the battery voltage is low by the notification unit, and then receive the transmission instruction.

The transmission control unit may cause the transmission of the still image data during a transmission period for the moving image data.

The transmission control unit may suppress transmission of the moving image data.

The transmission control unit may temporally stop and transmit the still image data.

The endoscope scope according to claim 1, wherein the instruction information prohibits the other wireless device using the same frequency from performing wireless transmission for a time required to transmit two or more packets of the still image data.

The endoscope scope wirelessly transmits moving image data and still image data generated by photographing a subject to a processor that receives the moving image data and the still image data and displays a moving image and a still image. The endoscope scope includes: a generation unit that generates the moving image data and the still image data; a reception unit that receives a transmission instruction for the still image data; a transmission unit that wirelessly transmits the still image data, the moving image data, and instruction information to instruct prohibition of wireless transmission by another wireless device; and a transmission control unit that causes the instruction information to be transmitted and then the still image data to be transmitted when receiving the transmission instruction.

The endoscope scope may further include: a voltage detection unit that detects battery voltage. The transmission control unit may receive the transmission instruction, and cause the instruction information to be transmitted and then the still image data to be transmitted when the battery voltage detected by the voltage detection unit is less than a predetermined value.

The reception unit may further receive a power-off instruction. The transmission control unit receives the transmission instruction and the power-off instruction, and causes the instruction information to be transmitted and then the still image data to be transmitted when the still image data remains in the endoscope scope.

The endoscope scope may further include: a device detection unit that detects the other wireless device. The transmission control unit may receive the transmission instruction, and cause the instruction information to be transmitted and then the still image data to be transmitted when the other wireless device is detected.

The endoscope scope may further include: a voltage detection unit that detects battery voltage; and a notification unit that notifies a processor that the battery voltage is low when the battery voltage detected by the voltage detection unit becomes less than a predetermined value. The transmission control unit may cause the instruction information to be transmitted and then the still image data to be transmitted when the transmission control unit is notified that the battery voltage is low by the notification unit, and then receive the transmission instruction.

The transmission control unit may cause the transmission of the still image data during a transmission period for the moving image data.

The transmission control unit may suppress transmission of the moving image data.

The transmission control unit may temporally stop and transmit the still image data.

The instruction information may prohibit the other wireless device using the same frequency from performing wireless transmission for a time required to transmit two or more packets of the still image data.

### Effect of the Invention

According to the present invention, when a transmission instruction for still image data is received, an endoscope scope transmits instruction information to instruct prohibition of wireless transmission by another wireless device and then transmits the still image data. The other wireless device receiving the instruction information stops wireless transmission, such that retransmission of the still image data caused by interference is suppressed. For this reason, it is possible to reduce a decrease in the battery capacity caused by retransmission of a still image in the endoscope scope, and transmit the still image data.

### BRIEF DESCRIPTION OF THE DRAWINGS

FIG. 1 is a block diagram illustrating a configuration of a scope in accordance with a first preferred embodiment of the present invention.
FIG. 2 is a block diagram illustrating a configuration of a processor in accordance with the first preferred embodiment of the present invention.
FIG. 3 is a reference diagram illustrating a situation in which moving image data is transmitted in accordance with the first preferred embodiment of the present invention.
FIG. 4 is a reference diagram illustrating a situation in which moving image data and still image data are transmitted in accordance with the first preferred embodiment of the present invention.
FIG. 5 is a graph illustrating the relationship between battery capacity and battery voltage in accordance with the first preferred embodiment of the present invention.
FIG. 6 is a reference diagram illustrating a situation in which moving image data and still image data are transmitted in accordance with the first preferred embodiment of the present invention.
FIG. 7 is a reference diagram illustrating battery voltage and setting value in accordance with the first preferred embodiment of the present invention.
FIG. 8 is a flow chart illustrating a procedure of an operation of the scope in accordance with the first preferred embodiment of the present invention.
FIG. 9 is a reference diagram illustrating a situation in which moving image data and still image data are transmitted in accordance with the first preferred embodiment of the present invention.
FIG. 10 is a reference diagram illustrating a situation in which moving image data and still image data are transmitted in accordance with the first preferred embodiment of the present invention.
FIG. 11 is a reference diagram illustrating a situation in which moving image data and still image data are transmitted in accordance with the first preferred embodiment of the present invention.
FIG. 12 is a flow chart illustrating a procedure of an operation of the scope in accordance with a second preferred embodiment of the present invention.
FIG. 13 is a flow chart illustrating a procedure of an operation of the scope in accordance with a third preferred embodiment of the present invention.
FIG. 14 is a flow chart illustrating a procedure of an operation of the scope in accordance with a fourth preferred embodiment of the present invention.

### BEST MODE FOR CARRYING OUT THE INVENTION

Hereinafter, preferred embodiments of the present invention will be described with reference to the drawings. Based on the disclosure herein, it is apparent to those of ordinary skill in the art that the following description of the preferred embodiments of the present invention is provided only for the purpose of illustrating the invention as defined by the appended claims and their equivalents in detail and not for the purpose of limiting them.

### First Preferred Embodiment

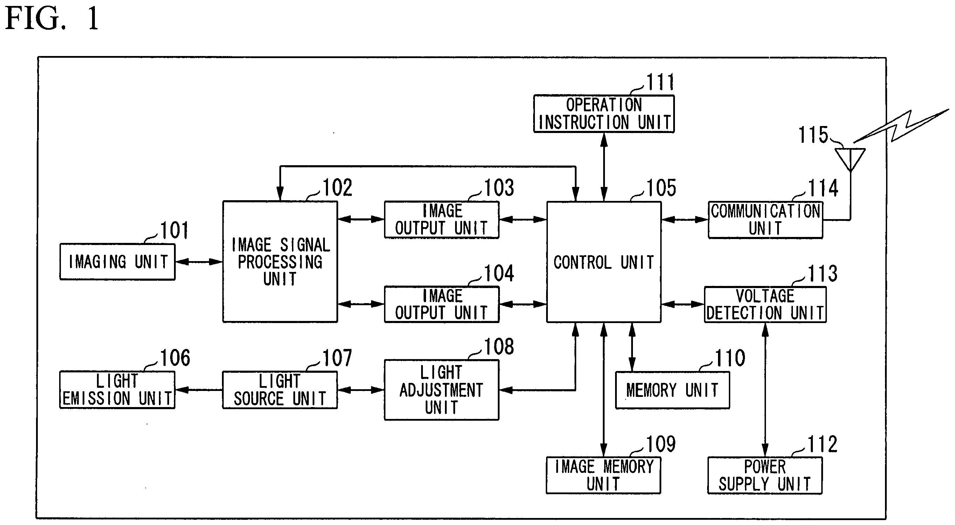

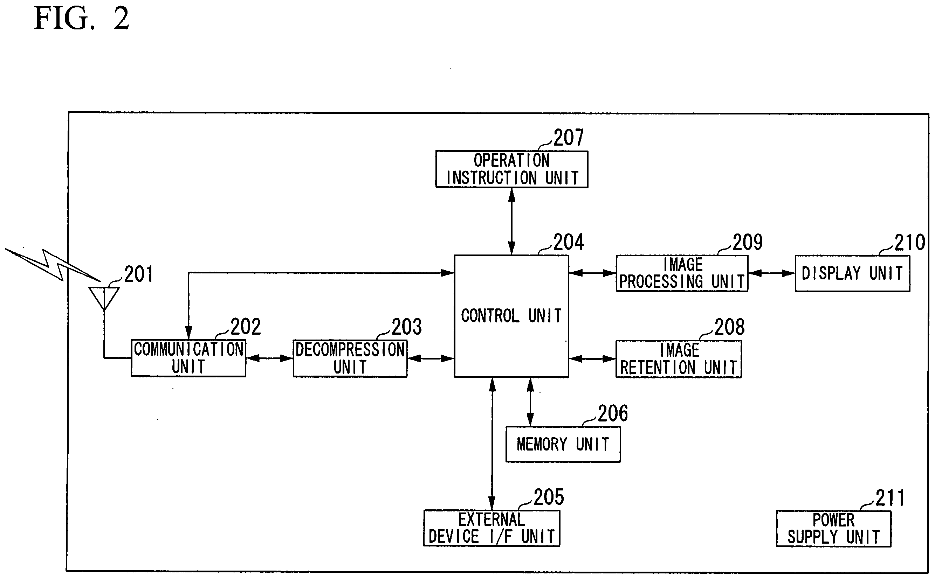

First, a first preferred embodiment of the present invention will be described. An endoscope scope (hereinafter referred to as a "scope") in accordance with the first preferred embodiment is configured to transmit image data to a processor that receives the image data, the scope and the processor being wirelessly connected to each other forming a wireless endoscope system. FIG. 1 shows a configuration of the scope, and FIG. 2 shows a configuration of the processor.

As shown in FIG. 1, the scope includes an imaging unit 101, an image signal processing unit 102, image output units 103 and 104, a control unit 105, a light emission unit 106, a light source unit 107, a light adjustment unit 108, an image memory unit 109, a memory unit 110, an operation instruction unit 111, a power supply unit 112, a voltage detection unit 113, a communication unit 114, and an antenna 115.

The imaging unit 101 includes a charge-coupled device (CCD) that photographs a subject, and an analog/digital converter (ADC) that converts an analog signal output from the CCD into a digital signal. The image signal processing unit 102 generates image data from the digital data output from the imaging unit 101. The image data is data of a moving or still image. The image output unit 103 performs lossy compression on the image data processed by the image signal processing unit 102 and outputs the compressed image data. The image output unit 104 compresses the image data processed by the image signal processing unit 102 at a lower compression ratio than that of the image output unit 103 and outputs the compressed image data, or outputs the image data without compression. The control unit 105 performs a variety of control operations.

The light emission unit 106 irradiates light to a coelom. The light source unit 107 includes a light-emitting diode (LED) and the like that supply light to the light emission unit 106. The light adjustment unit 108 adjusts an amount of light in the coelom. The image memory unit 109 stores image data output from each image output unit. The memory unit 110 stores a variety of programs and parameters. The operation instruction unit 111 includes a control lever and various switches (a power button, a channel button, and the like) of the scope to receive a freeze instruction, a power-off instruction, and the like from a user. The power supply unit 112 includes a battery that supplies power. The voltage detection unit 113 detects a battery voltage and outputs a control signal to the control unit 105. The communication unit 114 wirelessly performs data communication with the processor through the antenna 115. The antenna 115 performs wireless transmission and reception with the processor.

The processor includes an antenna 201, a communication unit 202, a decompression unit 203, a control unit 204, an external device interface (I/F) unit 205, a memory unit 206, an operation instruction unit 207, an image retention unit 208, an image processing unit 209, a display unit 210, and a power supply unit 211.

The antenna 201 performs wireless transmission and reception with the scope. The communication unit 202 performs data communication with the scope. The decompression unit 203 decompresses compression data received by the communication unit 202 to generate image data. When uncompressed image data is received by the communication unit 202, the decompression unit 203 does not perform decompression. The control unit 204 performs a variety of control operations. The external device I/F unit 205 is an interface capable of connecting to an external medium and an external device.

The memory unit 206 stores a variety of programs and parameters. The operation instruction unit 207 includes a variety of switches. The image retention unit 208 holds the image data decompressed by the decompression unit 203 or the uncompressed data. The image processing unit 209 processes the image data held in the image retention unit 208. The display unit 210 displays an image based on the image data processed by the image processing unit 209. The power supply unit 211 supplies power.

Next, transmission of a moving image and transmission of a still image will be described. In the scope, when data of a photographed moving image is transmitted, the communication unit 114 packetizes moving image data and performs a predetermined modulation process on the packets. Subsequently, the communication unit 114 performs carrier sensing before transmission of the packets. When there is no carrier from another device (a device using Institute of Electrical and Electronics Engineers (IEEE) 802.11b or the like), the communication unit 114 performs data transmission. On the other hand, when a carrier from another device is detected, the communication unit 114 generates a random number within a range defined by a contention window, and performs a retransmission process after waiting for an amount of time obtained by multiplying the generated number by a unit time (slot time).

In the processor, the antenna 201 receives a radio wave radiated from the scope, and the communication unit 202 reproduces data. When an error does not occur as a result of reproducing data, the processor returns an (acknowledgement) ACK to the scope. Moving image data is transmitted according to frame periods. When an error occurs in a packet, there is no ACK from the processor side. Thus, the scope determines that the transmission has failed, and performs retransmission. The retransmission of moving image data is performed within a moving image frame period. When the retransmission is not completed within the moving image frame period, the moving image data is discarded, and newly photographed moving image data is transmitted.

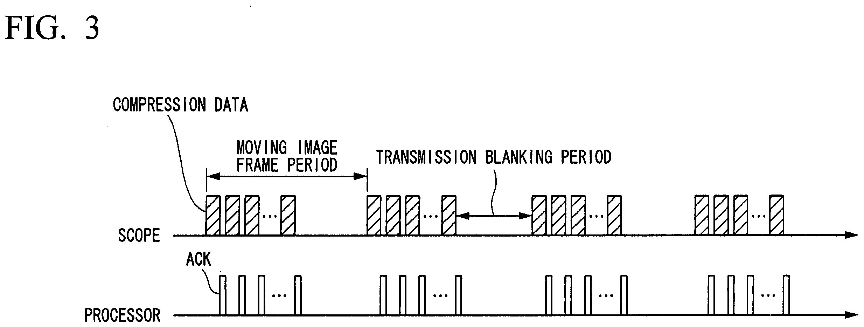

FIG. 3 illustrates a situation in which moving image data is transmitted. Within a moving image frame period, packets of compression data are repeatedly transmitted from the scope to the processor. When each packet is received by the processor, an ACK is transmitted from the processor to the scope. When the transmission of compression data corresponding to one frame is completed, it becomes a transmission blanking period until transmission of compression data of the next frame is started, and transmission of compression data is stopped.

When the operation instruction unit 111 of the scope receives a freeze instruction from a user while moving image data is transmitted (during a moving image frame period), the operation instruction unit 111 outputs a signal denoting the freeze instruction. The control unit 105 detecting the signal instructs the image signal processing unit 102 to perform output to the image output unit 104 in order to generate high-quality still image data. Thereby, image data processed by the image signal processing unit 102 is output to the image output unit 104, and still image data processed by the image output unit 104 is stored in the image memory unit 109.

Upon transmission of still image data, the communication unit 114 stops transmission of moving image data and transmits still image data packets to the processor through the antenna 115 after carrier sensing. The processor stores the received still image data in the image retention unit 208. Thereby, the still image data is held in the processor.

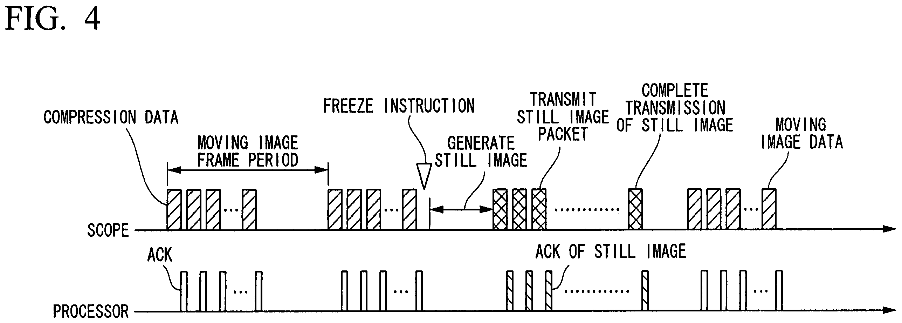

FIG. 4 illustrates a situation in which still image data is transmitted. If a freeze instruction is generated while moving image data is transmitted, transmission of compression data is stopped after transmission of compression data in a moving image frame period in which the freeze instruction has been generated is completed. Subsequently, still image data is generated, and packets of the still image data are repeatedly transmitted from the scope to the processor. When each packet is received by the processor, an ACK is transmitted from the processor to the scope. When the transmission of the still image data is completed, transmission of moving image data is resumed.

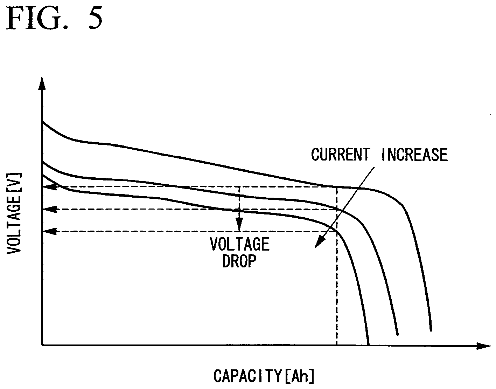

The scope is driven by a battery power supply. FIG. 5 illustrates the relationship between battery capacity and battery voltage. As illustrated in FIG. 5, when current consumption of the scope increases, the battery voltage is lowered even at the same battery capacity. When a still image data packet is damaged by interference caused by a wireless device based on IEEE802.11b and the like, the processor cannot receive the packet normally and thus cannot return ACK, and the scope retransmits the still image data. Since still image data does not require a real-time processing, the scope constantly attempts retransmission while interference is present. If retransmission is repeated, current consumption per unit time increases. For this reason, in the discharge curve shown in FIG. 5 as an example, when battery capacity is reduced, the battery voltage becomes lower than a voltage required to operate the scope. Then, the control unit 105 of the scope determines that the battery has been discharged and shuts down the scope, and the scope cannot transmit still image data.

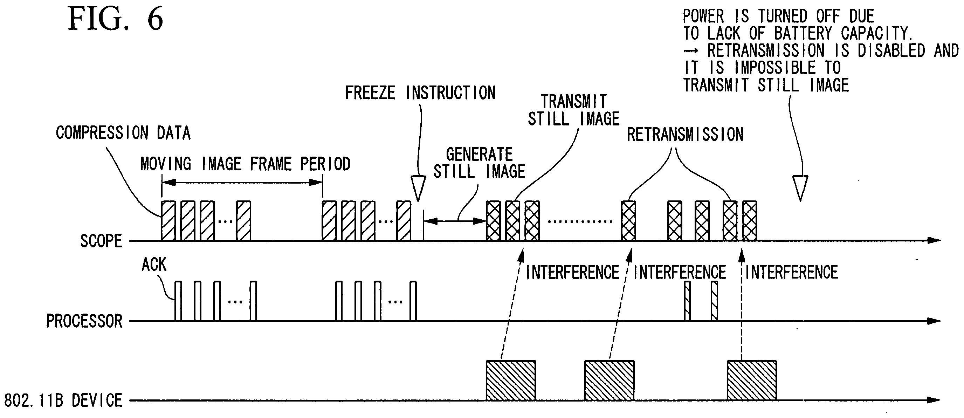

FIG. 6 illustrates a situation in which still image data is transmitted when battery voltage is lowered. If interference is caused by a nearby wireless device based on IEEE802.1 1b and the like when the scope transmits packets of still image data, the processor cannot receive the packets, and no ACK is returned. For this reason, the scope retransmits the packets of the still image data. When the battery voltage becomes lower than a voltage required to operate the scope due to the retransmission of the packets, the scope is shut down and cannot transmit the still image data.

To suppress the above-described prohibition on transmitting still image data, the first preferred embodiment employs the voltage detection unit 113 that checks whether or not battery voltage is enough to transmit the still image data upon transmission, and also a solution means of transmitting a clear-to-send (CTS) packet (instruction information) before still image data packets are transmitted if the voltage value detected by the voltage detection unit 113 is lower than a predetermined value.

A CTS packet serves to notify another wireless device using the same frequency as the wireless endoscope system of a transmission time corresponding to one or more still image data packets. In other words, the CTS packet serves to instruct prohibition of wireless transmission by other wireless devices. The wireless devices receiving the CTS packet wait for data transmission for the transmission time set by the CTS packet. The set time is referred to as a network allocation vector (NAV) period. The scope transmits still image data during the NAV period, thereby enabling communication in which retransmission is suppressed. Thus, even when battery capacity becomes low, it is possible to transmit the still image data in a short time. Then, current consumption per unit time can be controlled, such that the still image data can be stored in the processor. In order to suppress retransmission of still image data caused by interference and transmit the still image data in as short a time as possible, it is preferable to notify another wireless device of a transmission time corresponding to two or more packets of the still image data using a CTS packet.

When IEEE802.11g is applied to data communication of the wireless endoscope system, the corresponding data is an orthogonal frequency division multiplexing (OFDM) frame, and a wireless device based on IEEE802.11b cannot recognize the OFDM frame. In this case, the wireless device based on IEEE802.11b may consider the OFDM frame to be an interference wave from another system and start transmission even if a wireless device based on IEEE802.11g is performing transmission. As a result, a frame collision occurs, causing many retransmission operations. To avoid this problem, a procedure in which the wireless device based on IEEE802.11g transmits a CTS frame (Clear to Send frame) to its own station before transmitting the OFDM frame to suppress transmission of the wireless device based on IEEE802.11b has been provided.

However, when a CTS packet is transmitted prior to all transmission data, communication quality of its own system can be ensured, but data transmission of nearby wireless devices based on IEEE802.11b is suppressed, which may exert great influence on other wireless systems. In the first preferred embodiment, the scope attaches a CTS packet to still image data and transmits the still image data only when battery capacity is insufficient, and thus can coexist with other wireless devices when the battery capacity is sufficient.

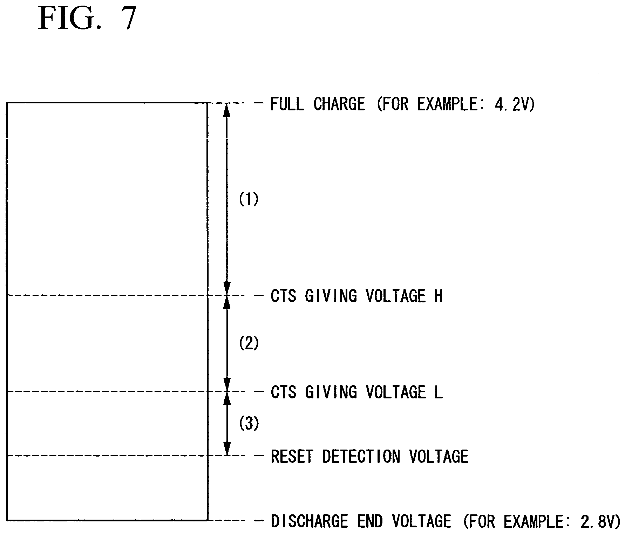

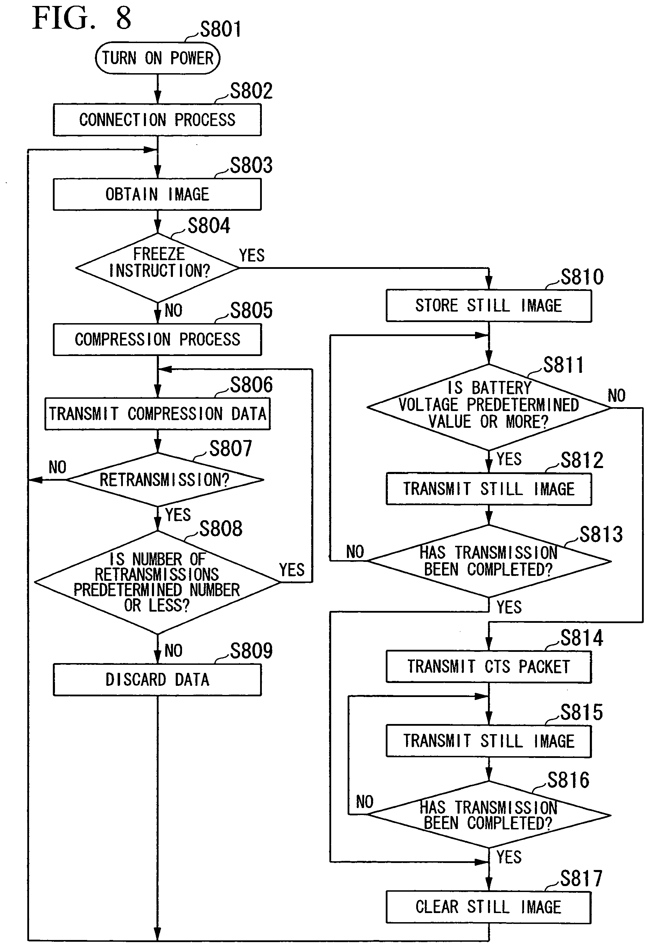

Next, operation of the scope will be described with reference to FIG. 7 and FIG. 8. Steps S801 to S809 of FIG. 8 correspond to the flow of general moving image data transmission. When the power of the scope is turned on (step S801), the scope performs a process for connecting to the processor (step S802). Subsequently, the imaging unit 101 generates a digital signal, and the image signal processing unit 102 generates image data from the digital signal (step S803). Subsequently, the control unit 105 determines whether or not there is a freeze instruction based on a signal from the operation instruction unit 111 (step S804).

When there is no freeze instruction, the control unit 105 instructs the image signal processing unit 102 to perform output to the image output unit 103. Thereby, the image data processed by the image signal processing unit 102 is output to the image output unit 103. The image output unit 103 performs a compression process on the input image data (step S805). The compressed image data (compression data) is stored in the image memory unit 109, and then output to the communication unit 114. The communication unit 114 generates packets of the compression data, and transmits the packets to the processor through the antenna 115 (step S806).

After transmission of the packets, the communication unit 114 properly receives ACKs from the processor, and notifies the control unit 105 that ACKs have been received. When the transmission of one packet is finished, the control unit 105 checks whether or not there is an ACK corresponding to the packet, and determines whether or not to retransmit the data (step S807). When ACKs corresponding to all the packets are received, and retransmission of the data is not required, the process from step S803 is performed again on the next frame. On the other hand, when there is a packet for which the ACK has not been received, the control unit 105 determines the number of retransmissions (step S808).

When the number of retransmissions is a predetermined number or less, the packet is transmitted to the processor again in step S806. On the other hand, when the number of retransmissions exceeds the predetermined number, the compression data of the current frame is discarded (step S809). Subsequently, the process from step S803 is performed again on the next frame.

When it is determined in step 804 that there is a freeze instruction, the control unit 105 instructs the image signal processing unit 102 to perform output to the image output unit 104. Thereby, the image data (still image data) processed by the image signal processing unit 102 is output to the image output unit 104. The image output unit 104 performs a compression process on the input still image data and outputs the compressed still image data, or outputs the input still image data without compression. The still image data output from the image output unit 104 is stored in the image memory unit 109 (step S810).

The voltage detection unit 113 repeatedly detects battery voltage of the power supply unit 112 and notifies the control unit 105 of the battery voltage. The control unit 105 compares the battery voltage with a setting value shown in FIG. 7, thereby determining whether or not the battery voltage is the predetermined value or more (step S811). When the battery voltage is the predetermined value or more (e.g., when the battery voltage is in an area of (1) of FIG. 7), the control unit 105 outputs the still image data stored in the image memory unit 109 to the communication unit 114. The communication unit 114 generates packets of the still image data and transmits the packets to the processor through the antenna 115 (step S812). Also, an ACK is also received when still image data is transmitted. Thus, when no ACK is received, the still image data is retransmitted, but this operation is omitted in FIG. 8.

Subsequently, the control unit 105 determines whether or not transmission of the still image data has been completed (step S813). When the transmission of the still image data has not been completed, the process from step S811 is performed again. On the other hand, when the transmission of the still image data has been completed, the control unit 105 discards the still image data stored in the image memory unit 109 (step S817). Subsequently, the process from step S803 is performed again.

When it is determined in step S811 that the battery voltage is less than the predetermined value (e.g., the battery voltage is in an area of (2) of FIG. 7), the control unit 105 instructs the communication unit 114 to transmit a CTS packet. The communication unit 114 transmits the CTS packet to nearby wireless devices (802.11b devices) (step S814). Thereby, the nearby wireless devices are notified of a NAV period for the scope to perform transmission. Subsequently, the control unit 105 outputs the still image data stored in the image memory unit 109 to the communication unit 114. The communication unit 114 generates packets of the still image data, and transmits the packets to the processor through the antenna 115 (step S815).

Subsequently, the control unit 105 determines whether or not the transmission of the still image data has been completed (step S816). When the transmission of the still image data has not been completed, the process from step S815 is performed again. On the other hand, when the transmission of the still image data has been completed, the control unit 105 discards the still image data stored in the image memory unit 109 (step S817). Subsequently, the process from step S803 is performed again.

As described above, after the transmission of the CTS packet, the scope transmits the still image data during the NAV period in which other wireless devices wait for transmission, and thus can transmit the still image data with interference of the other wireless devices suppressed.

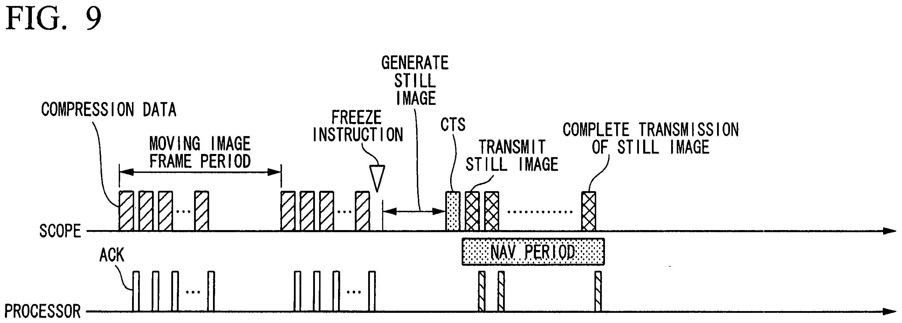

FIG. 9 illustrates a situation in which still image data is transmitted when battery voltage has been lowered. The scope checks battery voltage when transmitting packets of still image data. When the battery voltage is less than a predetermined value, the scope transmits a CTS packet, and then transmits the packets of the still image data during a NAV period designated using the CTS packet. Nearby wireless devices receive the CTS packet, and thus stop data transmission during the NAV period.

In the description above, transmission of moving image data is temporarily stopped to transmit still image data, but it is possible to transmit the still image data while transmitting the moving image data. In this case, the still image data is transmitted during a transmission blanking period shown in FIG. 3.

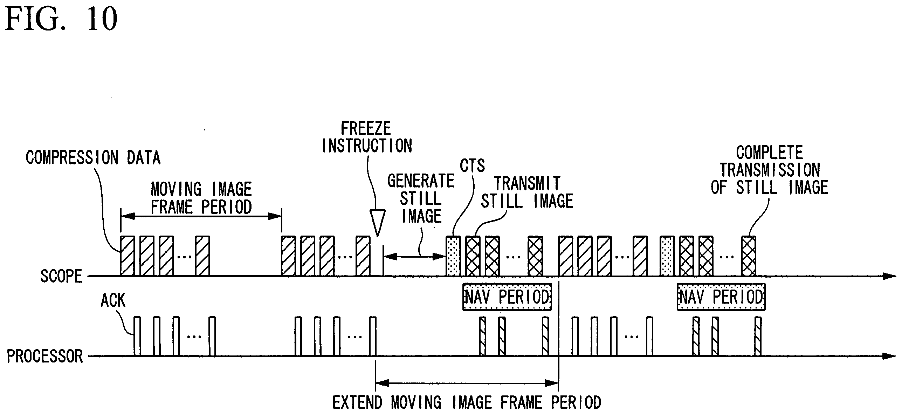

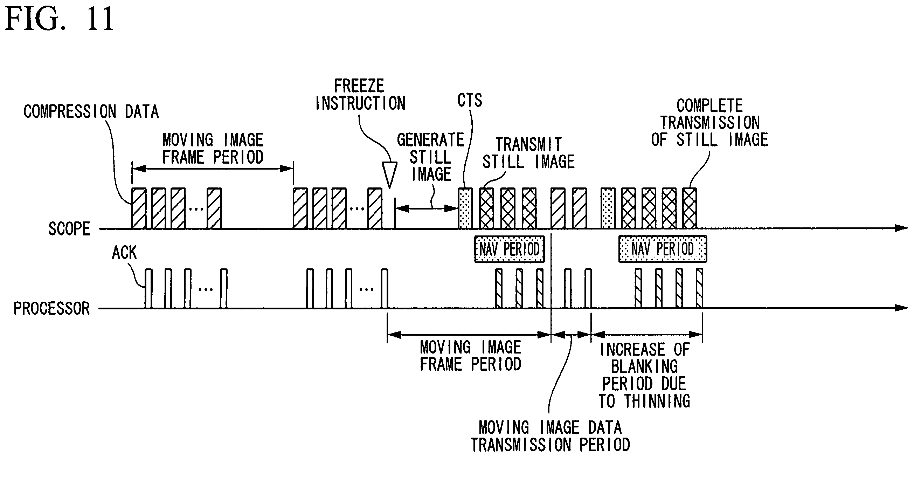

FIG. 10 and FIG. 11 illustrate situations in which still image data is transmitted during a transmission blanking period for moving image data. In FIG. 10, frames of moving image data typically including 30 frames per seconds are thinned out and still image data is transmitted. Although the amount of moving image data corresponding to one frame does not change, a blanking period for moving image data transmission lengthens, and thus the transmission time of the still image data can be increased. Thereby, it is possible to efficiently transmit the still image data while transmitting the moving image data.

In FIG. 11, the amount of data corresponding to one frame is reduced (data thinning) without changing the number of frames of moving image data to reduce transmission time of the moving image data and lengthen a blanking period of moving image transmission. Thereby, a transmission time of still image data can be increased, and still image data can be efficiently transmitted. To reduce the amount of data, a method of increasing a compression rate, a method of lowering a resolution of imaging data, and the like are generally used.

As described above, the scope in accordance with the first preferred embodiment receives a transmission instruction for still image data caused by a freeze instruction from a user, and transmits a CTS packet and then the still image data when battery voltage is less than a predetermined value. Other wireless devices receiving the CTS packet stop wireless transmission, and thereby retransmission of the still image data caused by interference is suppressed. For this reason, a decrease in battery capacity caused by retransmission in the scope is reduced, such that still image data can be transmitted.

### Second Preferred Embodiment

Next, a second preferred embodiment of the present invention will be described. An endoscope scope in accordance with the second preferred embodiment has the same configuration as the first preferred embodiment. In the first preferred embodiment, when there is a freeze instruction while moving image data is transmitted, the transmission of the moving image data is stopped to transmit still image data. However, in the second preferred embodiment, even when there is a freeze instruction, transmission of moving image data is not stopped but performed, and then (after the necessity to transmit the moving image data is removed) still image data is transmitted.

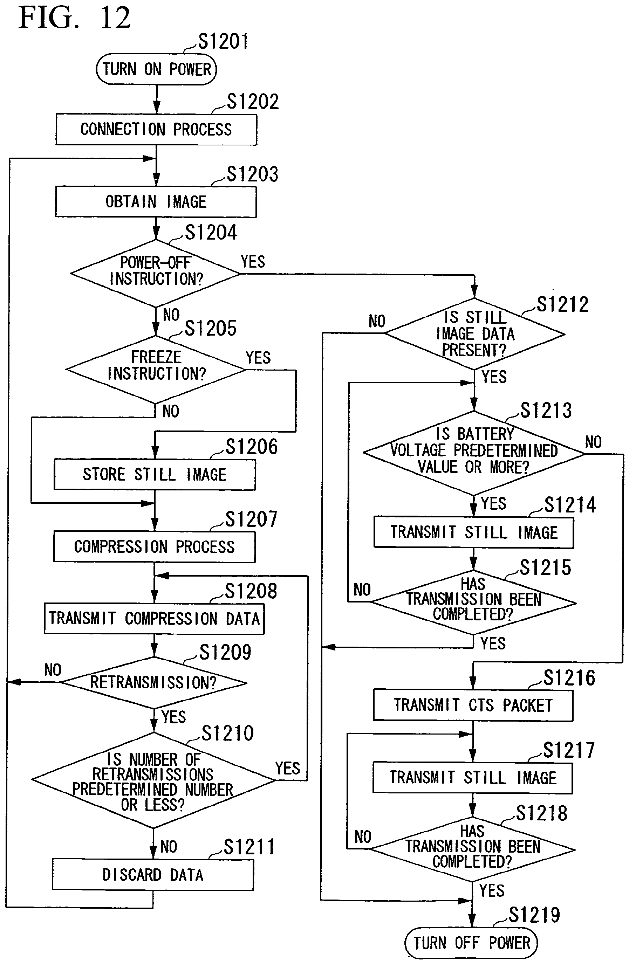

Operation of the scope in accordance with the second preferred embodiment will be described below with reference to FIG. 12. Steps S1201 to S1203 are the same as steps S801 to S803 of FIG. 8. After step S1203, the control unit 105 determines whether or not there is an instruction to turn off the power based on a signal from the operation instruction unit 111 (step S1204).

When there is no instruction to turn off the power, the control unit 105 determines whether or not there is a freeze instruction based on the signal from the operation instruction unit 111 (step S1205). When there is no freeze instruction, the process proceeds to step S1207. On the other hand, when there is a freeze instruction, the control unit 105 instructs the image signal processing unit 102 to perform output to the image output unit 104. Thereby, image data (still image data) processed by the image signal processing unit 102 is output to the image output unit 104. The image output unit 104 performs a compression process on the input still image data and outputs the compressed still image data, or outputs the input still image data without compression. The still image data output from the image output unit 104 is stored in the image memory unit 109 (step S1206). After step S1206, the process proceeds to step 1207. Steps S1207 to S1211 are the same as steps S805 to S809 of FIG. 8.

When it is determined in step S1204 that there is an instruction to turn off the power, the control unit 105 determines whether or not still image data has been stored in the image memory unit 109 (step S1212). When the still image data has been stored in the image memory unit 109, the process proceeds to step S1213. Steps S1213 to S1218 are the same as steps S811 to S816 of FIG. 8. When it is determined in step S1212 that no still image data has been stored in the image memory unit 109, or it is determined in step S1215 or S1218 that transmission of the still image data has been completed, the power is turned off (step S1219).

As described above, the scope in accordance with the second preferred embodiment receives a transmission instruction for still image data caused by a freeze instruction and an instruction to turn off the power, and transmits a CTS packet and then the still image data when battery voltage is less than a predetermined value and the still image data remains in the scope. Thereby, even when battery voltage is lowered, a moving image is not stopped after a freeze instruction and a user can continue observation. Also, the scope can transmit still image data at the same time.

### Third Preferred Embodiment

Next, a third preferred embodiment of the present invention will be described. An endoscope scope in accordance with the third preferred embodiment has the same configuration as the first preferred embodiment. In the first and second preferred embodiments, when a reduction of battery voltage is detected, a CTS packet is transmitted, and then still image data packets are transmitted. However, in the third preferred embodiment, when battery voltage is lowered, still image data is transmitted more efficiently.

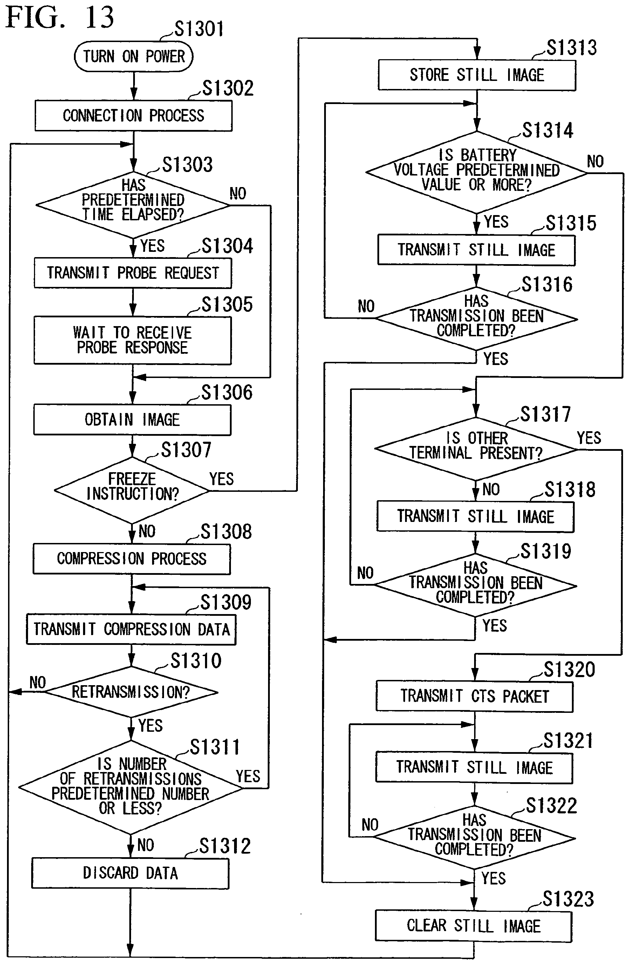

Operation of the scope in accordance with the third preferred embodiment will be described below with reference to FIG. 13. When the power of the scope is turned on (step S1301), the scope performs a process for connecting to the processor (step S1302). Subsequently, the control unit 105 transmits a last probe request and then determines whether or not a predetermined time has elapsed (step S1303).

When the predetermined time has not elapsed, the process proceeds to step S1306. On the other hand, when the predetermined time has elapsed, the control unit 105 instructs the communication unit 114 to transmit a probe request. The communication unit 114 transmits a probe request to nearby wireless devices (step S1304). Also when operation is performed for the first time after the power is turned on, the process proceeds to step S1304. Subsequently, the scope performs an operation of waiting for a probe response, which is a response to the probe request (step S1305). When the communication unit 114 receives the probe response during the waiting operation, the communication unit 114 notifies the control unit 105 of the reception of the probe response. By receiving the probe response, it is possible to know the presence of the nearby other wireless devices (terminals).

Subsequently, the process proceeds to step S1306. Steps S1306 to S1312 are the same as steps S803 to S809 of FIG. 8. Also, when it is determined in step S1307 that there is a freeze instruction, the process proceeds to step S1313. Steps S1313 to S1316 are the same as steps S810 to S813 of FIG. 8.

When it is determined in step S1314 that battery voltage is less than a predetermined value, the control unit 105 determines whether or not another wireless device is present based on the result of receiving the probe response in step S1305 (step S1317). When the probe response is received, there is another wireless device, and thus the process proceeds to step S1320. Steps S1320 to S1322 are the same as steps S814 to S816 of FIG. 8. On the other hand, when no probe response is received, the control unit 105 determines that there is no other wireless device, and the scope transmits still image data to the processor like in step S1321 (step S1318). Subsequently, the control unit 105 determines whether or not the transmission of the still image data has been completed (step S1319). When the transmission of the still image data has not been completed, the process from step S1317 is performed again.

When it is determined in steps S1316, S1319, and S1322 that the transmission of the still image data has been completed, the control unit 105 discards the still image data stored in the image memory unit 109 (step S1323). Subsequently, the process from step S1303 is performed again.

As described above, the scope in accordance with the third preferred embodiment receives a transmission instruction for still image data caused by a freeze instruction, and transmits a CTS packet when battery voltage is less than a predetermined value and another wireless device is detected. Thereby, it is unnecessary to transmit a CTS packet many times, and the still image data can be efficiently transmitted.

### Fourth Preferred Embodiment

Next, a fourth preferred embodiment of the present invention will be described. An endoscope scope in accordance with the fourth preferred embodiment has the same configuration as the first preferred embodiment. In the fourth preferred embodiment, upon transmission of still image data, a transmission method reflecting an intention of a user is selected.

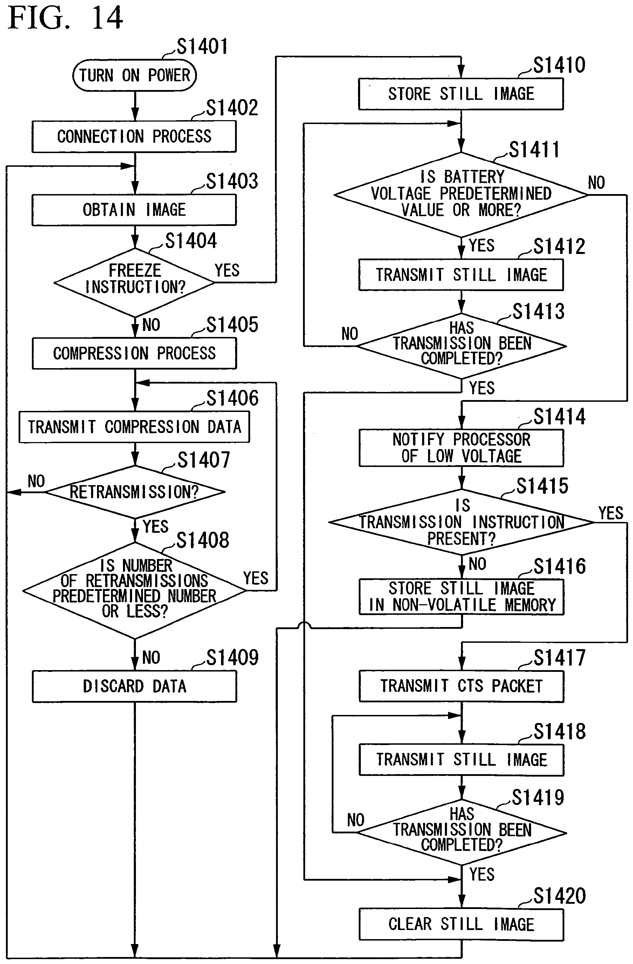

Operation of the scope in accordance with the fourth preferred embodiment will be described below with reference to FIG 14. Steps S1401 to S1413 are the same as steps S801 to S813 of FIG. 8. When it is determined in step S1411 that battery voltage is less than a predetermined value, the control unit 105 instructs the communication unit 114 to transmit notification information that notifies the processor that the battery voltage is low. The communication unit 114 transmits the notification information to the processor (step S1414).

The processor displays the information denoting that the battery voltage of the scope is low on a monitor based on the received notification information. A user determines whether or not to transmit still image data according to the information displayed on the monitor, and gives a transmission instruction by pressing a transmission switch present in the operation instruction unit 111 of the scope in a predetermined time when he/she determines to transmit the still image data.

After step S1414, the control unit 105 determines whether or not there is a transmission instruction based on a signal from the operation instruction unit 111 (step S1415). When there is a transmission instruction, the process proceeds to step S1417. Steps S1417 to S1420 are the same as steps S814 to S817 of FIG. 8. On the other hand, when there is no transmission instruction, still image data stored in the image memory unit 109 is stored in a non-volatile memory (step S1416). The non-volatile memory may be part of the image memory unit 109. As described in the second preferred embodiment, when there is an instruction to turn off the power, the still image data stored in the non-volatile memory may be read from the non-volatile memory and transmitted to the processor. After step S1416, the process from step S1403 is performed again.

As described above, the scope in accordance with the fourth preferred embodiment receives a transmission instruction for still image data caused by a freeze instruction, and notifies the processor that battery voltage is low when the battery voltage is less than a predetermined value. The processor receiving the notification notifies a user that the battery voltage is low. Thereby, the user can be notified that the battery voltage is low, and also it is possible to perform transmission of still image data reflecting an intention of the user.

When the battery capacity is low, and there is a large amount of still image data, transmission of the still image data may be disabled midway even after a CTS packet is transmitted. In this case, the scope notifies the processor that the still image data is not transmitted, and also stores the still image data in a non-volatile memory and the like. The processor notifies the user that the still image data is not transmitted, and also urges recharge of the battery. If the battery capacity is sufficient when the user turns on the power next time, the still image data may be transmitted.

While preferred embodiments of the present invention have been described and illustrated above, it should be understood that these are examples of the present invention and are not to be considered as limiting. Additions, omissions, substitutions, and other modifications can be made without departing from the scope of the present invention. Accordingly, the invention is not to be considered as being limited by the foregoing description, and is only limited by the scope of the claims.

### INDUSTRIAL APPLICABILITY

An endoscope scope of the present invention can reduce a decrease in battery capacity caused by retransmission of a still image in the endoscope scope, and transmit still image data.

### DESCRIPTION OF THE REFERENCE SYMBOLS

101 Imaging unit (Generation unit)
102 Image signal processing unit (Generation unit)
103 Image output unit
104 Image output unit
105 Control unit (Transmission control unit)
106 Light emission unit
107 Light source unit
108 Light adjustment unit
109 Image memory unit
110 Memory unit
111 Operation instruction unit (Reception unit)
112 Power supply unit
113 Voltage detection unit
114 Communication unit (Transmission unit, Device detection unit, Notification unit)
115 Antenna
201 Antenna
202 Communication unit
203 Decompression unit
204 Control unit
205 External device I/F unit
206 Memory unit
207 Operation instruction unit
208 Image retention unit
209 Image processing unit
210 Display unit
211 Power supply unit

## Claims

1. An endoscope comprising:
a generation unit (101) that is configured to photograph a subject and generate moving image data and still image data;
a reception unit (111) that is configured to receive a transmission instruction for the still image data from a user;
a voltage detection unit (113) that is configured to detect battery voltage;
a transmission unit (114) that is configured to wirelessly transmit the still image data and the moving image data to a processor, and to wirelessly transmit instruction information to another wireless device to instruct prohibition of wireless transmission by said another wireless device; and
a transmission control unit (105) that is configured to cause the transmission unit (114) to transmit the still image data when receiving the transmission instruction, and to cause the transmission unit (114) to transmit the instruction information before the transmission unit (114) transmits the still image data if the battery voltage detected by the voltage detection unit (113) is less than a predetermined value.

2. The endoscope according to claim 1, wherein
the reception unit (111) is further configured to receive a power-off instruction, and
the transmission control unit (105) is configured to receive the power-off instruction, and the transmission control unit (105) is configured to cause the transmission unit (114) to transmit the instruction information if the still image data remains in the endoscope.

3. The endoscope according to claim 1, further comprising:
a device detection unit (114) that is configured to detect the interfering wireless device,
wherein the transmission control unit (105) is configured to cause the transmission unit (114) to transmit the instruction information if interfering wireless device is detected.

4. The endoscope according to claim 1, further comprising:
a notification unit (114) that is configured to notify the processor, which is a transmission destination of the still image data, that the battery voltage is low if the battery voltage detected by the voltage detection unit (113) becomes less than a predetermined value,
wherein the transmission control unit (105) is configured to cause the transmission unit (114) to transmit the instruction information if the transmission control unit (105) receives the transmission instruction after the notification unit (114) notifies that the battery voltage is low.

5. The endoscope according to claim 1, wherein the transmission control unit (105) is configured to cause the transmission unit (114) to transmit the still image data during a transmission period for the moving image data.

6. The endoscope according to claim 1, wherein the transmission control unit (105) is configured to cause the transmission unit (114) to suppress transmission of the moving image data and to transmit the still image data.

7. The endoscope according to claim 1, wherein the transmission control unit (105) is configured to cause the transmission unit (114) to temporally-stop the transmission of the moving image data and to transmit the still image data.

8. The endoscope according to claim 1, wherein the transmission unit (114) is configured to transmit the instruction information for prohibiting the other wireless device using the same frequency from performing wireless transmission for a time required to transmit two or more packets of the still image data.

## Patentansprüche

1. Endoskop umfassend:
eine Erzeugungseinheit (101), welche konfiguriert ist, um ein Subjekt zu fotografieren und Bewegtbilddaten und Standbilddaten zu erzeugen;
eine Empfangseinheit (111), welche konfiguriert ist, um einen Sendebefehl für die Standbilddaten von einem Nutzer zu empfangen;
eine Spannungserkennungseinheit (113), welche konfiguriert ist, um eine Batteriespannung zu erkennen;
eine Sendeeinheit (114), welche konfiguriert ist, um die Standbilddaten und die Bewegtbilddaten kabellos zu einem Prozessor zu senden und um Befehlsinformation zu einer anderen kabellosen Vorrichtung kabellos zu senden, um ein Verbot von kabellosem Senden durch die andere kabellose Vorrichtung zu instruieren; und
eine Sendesteuereinheit (105), welche konfiguriert ist, um die Sendeeinheit (114) zu veranlassen, die Standbilddaten zu senden wenn der Sendebefehl empfangen wurde, und die Sendeeinheit (114) zu veranlassen, die Befehlsinformation zu senden, bevor die Sendeeinheit (114) die Standbilddaten sendet, wenn die durch die Spannungserkennungseinheit (113) erkannte Batteriespannung geringer als einen vorherbestimmten Wert ist.

2. Endoskop gemäß Anspruch 1, wobei
die Empfangseinheit (111) ferner konfiguriert ist, um ein Ausschaltbefehl zu empfangen, und
die Sendesteuereinheit (105) konfiguriert ist, um den Ausschaltbefehl zu empfangen, wobei die Sendesteuereinheit (105) konfiguriert ist, um die Sendeeinheit (114) zu veranlassen die Befehlsinformation zu senden, wenn die Standbilddaten in dem Endoskop verbleiben.

3. Endoskop gemäß Anspruch 1, ferner umfassend:
eine Vorrichtungserkennungseinheit (114), welche konfiguriert ist, um die störende kabellose Vorrichtung zu erkennen,
wobei die Sendesteuereinheit (105) konfiguriert ist, um die Sendeeinheit (114) zu veranlassen, die Befehlsinformation zu senden, wenn eine störende kabellose Vorrichtung erkannt wurde.

4. Endoskop gemäß Anspruch 1, ferner umfassend:
eine Benachrichtigungseinheit (114), welche konfiguriert ist, um den Prozessor zu benachrichtigen, welcher ein Sendeziel der Standbilddaten ist, dass die Batteriespannung niedrig ist, wenn die von der Spannungserkennungseinheit (113) erkannte Batteriespannung kleiner als ein vorherbestimmter Wert wird,
wobei die Sendesteuereinheit (105) konfiguriert ist, um die Sendeeinheit (114) zu veranlassen, die Befehlsinformation zu senden, wenn die Sendesteuereinheit (105) den Sendebefehl empfängt, nachdem die Benachrichtigungseinheit (114) benachrichtigt, dass die Batteriespannung niedrig ist.

5. Endoskop gemäß Anspruch 1, wobei die Sendesteuereinheit (105) konfiguriert ist, um die Sendeeinheit (114) zu veranlassen, die Standbilddaten während einer Sendeperiode der Bewegtbilddaten zu senden.

6. Endoskop gemäß Anspruch 1, wobei die Sendesteuereinheit (105) konfiguriert ist, um die Sendeeinheit (114) zu veranlassen, Senden der Bewegtbilddaten zu unterdrücken und die Standbilddaten zu senden.

7. Endoskop gemäß Anspruch 1, wobei die Sendesteuereinheit (105) konfiguriert ist, um die Sendeeinheit (114) zu veranlassen, das Senden der Bewegtbilddaten vorübergehend anzuhalten und die Standbilddaten zu senden.

8. Endoskop gemäß Anspruch 1, wobei die Sendeeinheit (114) konfiguriert ist, um die Befehlsinformation zu senden, um der anderen die gleiche Frequenz benutzenden kabellosen Vorrichtung zu verbieten, kabelloses Senden für eine Zeit auszuführen, die benötigt wird, um zwei oder mehr Pakete der Standbilddaten zu senden.

## Revendications

1. Endoscope comprenant :
une unité de génération (101) qui est configurée pour photographier un sujet et générer des données d'image mobile et des données d'image fixe ;
une unité de réception (111) qui est configurée pour recevoir, depuis un utilisateur, une instruction de transmission pour les données d'image fixe ;
une unité de détection de tension (113) qui est configurée pour détecter une tension de batterie ;
une unité de transmission (114) qui est configurée pour transmettre sans fil les données d'image fixe et les données d'image mobile à un processeur, et pour transmettre sans fil des informations d'instruction à un autre dispositif sans fil pour ordonner l'interdiction d'une transmission sans fil par ledit autre dispositif sans fil ; et
une unité de commande de transmission (105) qui est configurée pour amener l'unité de transmission (114) à transmettre les données d'image fixe lors de la réception de l'instruction de transmission, et pour amener l'unité de transmission (114) à transmettre les informations d'instruction avant que l'unité de transmission (114) transmette les données d'image fixe si la tension de batterie détectée par l'unité de détection de tension (113) est inférieure à une valeur prédéterminée.

2. Endoscope selon la revendication 1, dans lequel
l'unité de réception (111) est en outre configurée pour recevoir une instruction de mise hors tension, et
l'unité de commande de transmission (105) est configurée pour recevoir l'instruction de mise hors tension, et l'unité de commande de transmission (105) est configurée pour amener l'unité de transmission (114) à transmettre les informations d'instruction si les données d'image fixe restent dans l'endoscope.

3. Endoscope selon la revendication 1, comprenant en outre :
une unité de détection de dispositif (114) qui est configurée pour détecter le dispositif sans fil d'interférence,
dans lequel l'unité de commande de transmission (105) est configurée pour amener l'unité de transmission (114) à transmettre les informations d'instruction si le dispositif sans fil d'interférence est détecté.

4. Endoscope selon la revendication 1, comprenant en outre :
une unité de notification (114) qui est configurée pour notifier au processeur, qui est une destination de transmission des données d'image fixe, que la tension de batterie est faible si la tension de batterie détectée par l'unité de détection de tension (113) devient inférieure à une valeur prédéterminée,
dans lequel l'unité de commande de transmission (105) est configurée pour amener l'unité de transmission (114) à transmettre les informations d'instruction si l'unité de commande de transmission (105) reçoit l'instruction de transmission après que l'unité de notification (114) a notifié que la tension de batterie est faible.

5. Endoscope selon la revendication 1, dans lequel l'unité de commande de transmission (105) est configurée pour amener l'unité de transmission (114) à transmettre les données d'image fixe pendant une période de transmission pour les données d'image mobile.

6. Endoscope selon la revendication 1, dans lequel l'unité de commande de transmission (105) est configurée pour amener l'unité de transmission (114) à supprimer la transmission des données d'image mobile et à transmettre les données d'image fixe.

7. Endoscope selon la revendication 1, dans lequel l'unité de commande de transmission (105) est configurée pour amener l'unité de transmission (114) à arrêter temporairement la transmission des données d'image mobile et à transmettre les données d'image fixe.

8. Endoscope selon la revendication 1, dans lequel l'unité de transmission (114) est configurée pour transmettre les informations d'instruction pour interdire à l'autre dispositif sans fil utilisant la même fréquence de réaliser une transmission sans fil pendant un temps requis pour transmettre deux ou plus paquets de données d'image fixe.
